# EUROPEAN PATENT APPLICATION

(11) **EP 3 981 400 A1**
(43) Date of publication of application: **13.04.2022**
(21) Application number: 20822606.8
(22) Date of filing: 10.06.2020
(51) Int. Cl.: A61K 31/4184, A61K 31/519, A61K 9/48

(54) **ORAL CAPSULE AND PREPARATION METHOD THEREFOR**

(30) Priority: 10.06.2019 WO PCT/CN2019/090543
(71) Applicant: BeiGene Switzerland GmbH, 4051 Basel (CH)
(72) Inventor: GUO, Yuanjing, Beijing 102206 (CN); WANG, Yiping, Beijing 102206 (CN)
(74) Representative: Mewburn Ellis LLP
(86) International application number: PCT/CN2020/095353
(87) International publication number: WO 2020/249002

(57) **Abstract**

An oral capsule and a method for filling a capsule after directly mixing powders, the oral capsule comprising a com- position for the oral capsule and a capsule shell, the composition for the oral capsule comprising zanubrutinib, a filler, a disintegrant, a wetting agent, a glidant, a lubricant, and optionally a binder. The composition for the capsule is capable of obtaining satisfactory product stability, dissolution properties that meet bioavailability standards, a preparation process consistent with production equipment, and reasonable production costs. In addition, the method is a non-granulating process, which may simplify the overall process steps and reduce the impact of the preparation process on product bioavailability.

## Description

### Technical Field

The present disclosure belongs to the field of pharmaceutical preparations, and describes an oral capsule comprising a Bruton's Tyrosine Kinase (BTK) inhibitor, especially (S)-7-[4-(1-acryloylpiperidine)]-2-(4-phenoxyphenyl)-4,5,6,7-tetrahydropyrazolo[1,5-a]pyrimidine-3-carboxamide and a method for preparing the same.

### Background

International application WO2014173289A discloses a novel Bruton's Tyrosine Kinase (BTK), more specifically (S)-7-[4-(1-acryloylpiperidine)]-2-(4-phenoxyphenyl)-4,5,6,7-tetrahydropyrazolo[1,5-a]pyrimidine-3-carboxamide (INN: zanubrutinib), and its chemical structure is as follows:

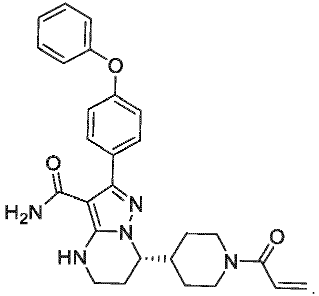

Zanubrutinib is a second-generation BTK inhibitor, which irreversibly inactivates tyrosine kinase by covalently binding to it. It is used alone or in combination with other drugs for the treatment of B lymphocyte tumor, including chronic lymphocytic leukemia/small lymphocytic lymphoma (CLL/SLL), mantle cell lymphoma (MCL), Waldenstrom's macroglobulinemia (WM), follicular lymphoma (FL), diffuse large B-cell lymphoma, non-germinal center subtype (non-GCB DLBCL), and the like.

An active pharmaceutical ingredient (API) of zanubrutinib is slightly hygroscopic. DSC results show that this compound, when melting, has a clear endothermic peak, with starting temperature and peak temperature of 139°C and 144°C, respectively, and it tends to have a sticking phenomenon. In addition, the zanubrutinib has pH-dependent solubility and belongs to a class II drug of the biopharmaceutical classification system. Therefore, it is necessary to maintain a good dissolution rate in the preparation.

The active pharmaceutical ingredient zanubrutinib used in the present disclosure has a smaller powder particle size after being pulverized, and therefore has poor fluidity. In addition, the active pharmaceutical ingredient zanubrutinib used in the present disclosure also has defects in respect of physical and chemical properties (high viscosity and poor fluidity). The formula of the capsule in the present disclosure can solve the above problems at reasonable production costs.

### Summary

In order to overcome the above problems of the active pharmaceutical ingredient zanubrutinib, the present disclosure provides a capsule formula and a method for directly encapsulating mixed powder into a capsule.

Specifically, the technical solution of the present disclosure is as follows:
In a first aspect of the present disclosure, an oral capsule is provided, comprising a composition for the oral capsule and a capsule shell, where the composition for the oral capsule comprises zanubrutinib, a filler, a disintegrant, a wetting agent, a glidant, and a lubricant.
In some embodiments, the composition for the oral capsule further comprises an optional binder.

Preferably, the zanubrutinib may be in any solid form, such as a crystal form (e.g., the crystal form A disclosed in WO2018033853A) or an amorphous form or a mixture of a crystal form and an amorphous form. Preferably, the zanubrutinib is a crystal form A, an amorphous form, or a mixture of a crystal form A and an amorphous form. A particle size of the zanubrutinib is less than or equal to 40 µm, and a mass percent of the zanubrutinib is from 20% to 70%, preferably from 20% to 50%, relative to a total mass of the composition for the oral capsule.

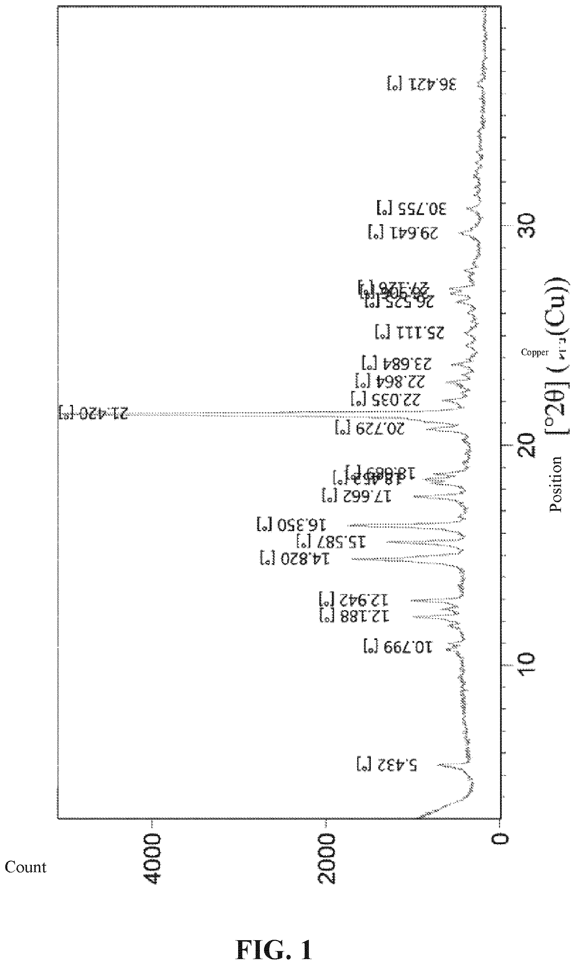

In some embodiments, an X-ray powder diffraction pattern of the crystal form A includes diffraction peaks having 2θ angle values independently selected from the group consisting of: about 14.8±0.2°, 15.6±02°, 16.4±0.2°, and 21.4±0.2°. In some embodiments, an X-ray powder diffraction pattern of the crystal form A includes diffraction peaks having 2θ angle values independently selected from the group consisting of: about 12.2±0.2°, 12.9±0.2°, 14.8±0.2°, 15.6±02°, 16.4±0.2°, and 21.4±02°. In some embodiments, an X-ray powder diffraction pattern of the crystal form A includes diffraction peaks having 2θ angle values independently selected from the group consisting of: about 12.2±0.2°, 12.9±0.2°, 14.8±0.2°, 15.6±02°, 16.4±02°, 17.7±0.2°, 18.5±0.2°, 20.7±0.2°, and 21.4±02°. In some embodiments, an X-ray powder diffraction pattern of the crystal form A is substantially consistent with that in **Fig. 1**.

Preferably, the filler is selected from the group consisting of starch, sucrose, microcrystalline cellulose, anhydrous calcium hydrogen phosphate, mannitol, lactose, pregelatinized starch, glucose, maltodextrin, cyclodextrin, cellulose, silicified microcrystalline cellulose, and any combination thereof; and a mass percent of the filler is from 20% to 90%, preferably from 30% to 80%, relative to the total mass of the composition for the oral capsule. An average particle size of the filler is preferably from 100 µm to 200 µm. More preferably, the average particle size of the filler is consistent with the average particle size of the active pharmaceutical ingredient, to ensure the product mixing uniformity, thereby contributing to the process scale-up.

More preferably, the filler is microcrystalline cellulose or a mixture of microcrystalline cellulose and colloidal silicon dioxide, or anhydrous calcium hydrogen phosphate is further added. Further preferably, the filler is microcrystalline cellulose; and a mass percent of the microcrystalline cellulose is from 30% to 80% relative to the total mass of the composition for the oral capsule.

Preferably, the disintegrant is selected from the group consisting of sodium carboxymethyl starch, low-substituted hydroxypropylcellulose, crospovidone, croscarmellose sodium, croscarmellose, methylcellulose, pregelatinized starch, sodium alginate, and any combination thereof; and a mass percent of the disintegrant is from 0.5% to 5%, preferably from 1% to 3%, relative to the total mass of the composition for the oral capsule. More preferably, the disintegrant is croscarmellose sodium.

Preferably, the wetting agent is sodium dodecyl sulfate; and a mass percent of the sodium dodecyl sulfate is from 0% to 5%, preferably from 0.5% to 1.0%, relative to the total mass of the composition for the oral capsule.

Preferably, the glidant is selected from the group consisting of powdery cellulose, magnesium trisilicate, colloidal silicon dioxide, talcum powder, and any combination thereof; and a mass percent of the glidant is from 0.1% to 20%, preferably from 0.1% to 0.5%, relative to the total mass of the composition for the oral capsule. More preferably, the glidant is colloidal silicon dioxide.

Preferably, the lubricant is selected from the group consisting of zinc stearate, glyceryl monostearate, glyceryl palmitostearate, magnesium stearate, sodium stearyl fumarate, and any combination thereof; and a mass percent of the lubricant is from 0.1% to 2%, preferably from 0.3% to 1%, relative to the total mass of the composition for the oral capsule. More preferably, the lubricant is magnesium stearate.

Preferably, the binder is selected from the group consisting of starch, hypromellose, polyvinylpyrrolidone, sodium carboxymethylcellulose, hydroxypropylcellulose, methylcellulose, ethylcellulose, gelatin, sucrose, and any combination thereof; and a mass percent of the binder is from 0 to 10%, preferably from 0 to 5%, relative to the total mass of the composition for the oral capsule. More preferably, the binder is hypromellose.

Preferably, the capsule shell is a gelatin capsule shell.

An intermediate of the capsule of the present disclosure has good fluidity, and is suitable for direct encapsulation after mixing without the need for granulation, thereby simplifying the overall process steps and reducing the impact of the direct process on the product bioavailability. Furthermore, the above mentioned composition for the capsule is characterized by satisfactory product stability, dissolution properties that meet bioavailability standards, a preparation process consistent with production equipment, and reasonable production costs.

In another aspect of the present disclosure, a method for preparing an oral zanubrutinib capsule is provided, including the following steps:
(1) pre-mixing zanubrutinib, a disintegrant, a wetting agent and a part of a filler to obtain a premix, and then sieving the premix to obtain a first mixture;
(2) sieving a glidant and the remaining part of the filler, and adding the sieved materials to the first mixture obtained in step (1) and mixing to obtain a second mixture;
(3) sieving a lubricant, adding the sieved lubricant to the second mixture obtained in step (2), and then mixing to obtain a final mixture; and
(4) encapsulating the final mixture obtained in step (3) into a capsule shell to obtain the oral capsule.

Preferably, the sieving is performed using a conical granulator.

Preferably, the pre-mixing in step (1) is performed at a revolving speed from 10 rpm to 25 rpm for 2 min to 10 min, and a sieve used for the sieving has a mesh size from 1.0 mm to 2.5 mm.

Preferably, the first mixture in step (1) is obtained by mixing the premix at a revolving speed from 10 rpm to 25 rpm for 40 min to 15 min.

Preferably, the mixing in step (2) is performed at a revolving speed from 10 rpm to 20 rpm for 3 min to 5 min.

Preferably, the sieving in step (2) is performed at a revolving speed from 550 rpm to 650 rpm, and a sieve used for the sieving has a mesh size from 1.0 mm to 2.5 mm.

Preferably, the mixing in step (3) is performed at a revolving speed from 10 rpm to 15 rpm for 3 min to 6 min, and a sieve used for the sieving has a mesh size from 35 mm to 45 mm.

In the method for preparing an oral capsule, when the mixing in steps (1) to (3) is insufficient, the active pharmaceutical ingredient will be non-uniformly distributed in the mixed powder; while when the mixing in steps (1) to (3) is excessive, the active pharmaceutical ingredient and auxiliary materials will be layered and isolated, thereby affecting the product quality.

Preferably, the method for preparing an oral zanubrutinib capsule includes the following steps:
(1) mixing the zanubrutinib, a disintegrant, a wetting agent and a part of a filler at a revolving speed of 20 rpm for 3 min to obtain a premix; sieving the premix through a sieve having a mesh size of 1.5 mm using a granulator at a revolving speed of 600 rpm; and mixing at a revolving speed of 20 rpm for 20 min or mixing at a revolving speed of 12 rpm for 35 min to obtain a first mixture;
(2) sieving a glidant and the remaining part of the filler through a sieve having a mesh size of 1.5 mm using a granulator at a revolving speed of 600 rpm; and adding the sieved materials to the first mixture obtained in step (1) and mixing at a revolving speed of 20 rpm for 5 min to obtain a second mixture;
(3) sieving a hard lubricant through a 40 mesh sieve, adding the sieved hard lubricant to the second mixture obtained in step (2), and then mixing at a revolving speed of 20 rpm for 5 min to obtain a final mixture; and
(4) encapsulating the final mixture obtained in step (3) into the capsule shell to obtain the oral capsule.

Preferably, the method for preparing an oral zanubrutinib capsule includes the following steps:
(1) mixing the zanubrutinib, a disintegrant, a wetting agent and a part of a filler at a revolving speed of 12 rpm for 5 min to obtain a premix; sieving the premix through a sieve having a mesh size of 1.9 mm or 2.0 mm using a granulator at a revolving speed of 600 rpm; and mixing at a revolving speed of 20 rpm for 20 min or mixing at a revolving speed of 12 rpm for 35 min to obtain a first mixture;
(2) sieving a glidant and the remaining part of the filler through a sieve having a mesh size of 1.9 mm or 2.0 mm using a granulator at a revolving speed of 600 rpm; and adding the sieved materials to the first mixture obtained in step (1) and mixing at a revolving speed of 12 rpm for 5 min to obtain a second mixture;
(3) sieving a hard lubricant through a 40 mesh sieve, adding the sieved hard lubricant to the second mixture obtained in step (2), and then mixing at a revolving speed of 12 rpm for 5 min to obtain a final mixture; and
(4) encapsulating the final mixture obtained in step (3) into the capsule shell to obtain the oral capsule.

Preferably, a loading amount of a mixing hopper is from 30% to 70% of the volume of the mixing hopper.

In thee above method of the present disclosure, mixed powder is directly encapsulated into a capsule. Therefore, the method has no granulation process, thereby simplifying the overall process steps and reducing the impact of the preparation process on the product bioavailability. Furthermore, the crystal form of an active pharmaceutical ingredient remains unchanged in the process.

In the above method of the present disclosure, an active pharmaceutical ingredient is firstly premixed with auxiliary materials, thereby effectively solving the problems of poor fluidity, easy agglomeration during storage, and difficulty in separate sieving of the active pharmaceutical ingredient, pulverizing agglomerates of the active pharmaceutical ingredient by sieving after premixing, and ultimately guaranteeing the content uniformity of the product. Furthermore, fully mixing the active pharmaceutical ingredient with the auxiliary materials portionwise can improve the product content and content uniformity. At the same time, reasonable preparation process parameters, such as only non-excessive lubricant mixing conditions, can ensure the product dissolution.

### Brief Description of the Drawings

FIG. 1 is an XRPD pattern of an active pharmaceutical ingredient zanubrutinib.

### Detailed Description

### Technical terms

Unless otherwise defined, all technical terms and scientific terms used in the present disclosure have the same meaning as commonly understood by those skilled in the art.

The terms "comprising," "including," or grammatical variations thereof used in the present disclosure mean that the composition and the method include the listed elements and do not exclude others.

Unless explicitly indicated otherwise, all ranges cited herein are inclusive; i.e., the ranges include the values of the upper limits and lower limits of the ranges and all values therebetween. For example, temperature ranges, percent, equivalent ranges, and the like described herein include the upper limits and lower limits of the ranges and any values in the continuous intervals therebetween.

The composition of the present disclosure includes a mixture of an active ingredient and other chemical ingredients.

The "optionally (optional)" of the present disclosure means that said item may be selected or may not be selected. For example, an optional binder means that the binder may be included or may not be included.

### Examples

The following examples may help those skilled in the art to more comprehensively understand the present disclosure, but do not impose any limitation on the present disclosure. The auxiliary materials are all commercially available.

### Example 1

### Preparation of oral zanubrutinib capsule

Formula for a single capsule:

| Zanubrutinib (crystal form A) | 80.00 mg |
|---|---|
| Microcrystalline cellulose | 263.80 mg |
| Croscarmellose sodium | 10.80 mg |
| Colloidal silicon dioxide | 1.8 mg |

| | |
|---|---|
| Sodium dodecyl sulfate | 1.8 mg |
| Magnesium stearate | 1.8 mg |
| Total amount | 360 mg |

### Preparation method:

(1) Zanubrutinib, sodium dodecyl sulfate, croscarmellose sodium and 60.28% microcrystalline cellulose were added into a mixing hopper, and then mixed at a revolving speed of 20 rpm for 3 min; the pre-mixed materials were sieved through a sieve having a mesh size of 1.5 mm using a conical sieving machine and using a granulator at a revolving speed of 600 rpm; and the sieved materials were transferred back to the mixing hopper, and then mixed at a revolving speed of 20 rpm for 20 minutes.
(2) The remaining part of microcrystalline cellulose and colloidal silicon dioxide were sieved together through a sieve having a mesh size of 1.5 mm using a granulator at a revolving speed of 600 rpm; and the sieved materials were transferred to a mixing hopper, and then mixed at a revolving speed of 20 rpm for 5 min.
(3) Magnesium stearate was sieved through a 40 mesh sieve, added to a mixing hopper, and then mixed at a revolving speed of 20 rpm for 5 min.
(4) The obtained mixed powder were encapsulated into an empty gelatin capsule shell to obtain an oral capsule with a filling amount of 360 mg.

The final mixed powder of Example 1 have good fluidity and uniform dispersion, satisfying the capsule filling.

In addition, the mixed powder were measured by XRPD in the study on the mixing process, and the results showed that the crystal form of zanubrutinib remained unchanged in the process.

**Cumulative dissolution rate (in vitro dissolution) test of a drug:** An in vitro dissolution experiment was carried out with a dissolution autosampler according to basket method in the Chinese Pharmacopoeia 0931 "dissolution" by setting the dissolution autosampler at a water bath temperature of 37+0.5 °C and at a revolving speed of100 rpm, and using 900 mL of 0.1N hydrochloric acid containing 0.5% sodium dodecyl sulfate as a dissolution medium. Samples were taken at 10 min, 15 min, 30 min, 45 min, and 60 min respectively. All samples passed through a 0.45 µm filter membrane, and were determined and analyzed according to the sample dissolution testing method. The oral zanubrutinib capsule in Example 1 has a dissolution rate (%)of more than 85% in the above dissolution medium in 30 min, satisfying the requirements for rapid release.

### Example 2

### Preparation of oral zanubrutinib capsule

Formula for a single capsule:

| Zanubrutinib (crystal form A) | 80.00 mg |
|---|---|
| Microcrystalline cellulose | 267.40 mg |
| Croscarmellose sodium | 7.20 mg |
| Colloidal silicon dioxide | 1.8 mg |
| Sodium dodecyl sulfate | 1.8 mg |

| | |
|---|---|
| Magnesium stearate | 1.8 mg |
| Total amount | 360 mg |

A target oral capsule was prepared with reference to a method similar to that in Example 1. The cumulative dissolution rate (in vitro dissolution) of the drug was determined with reference to the method in Example 1. The oral zanubrutinib capsule in Example 2 has a dissolution rate (%)of more than 85% in the above dissolution medium in 30 min.

### Example 3

### Preparation of oral zanubrutinib capsule

Formula for a single capsule:

| Zanubrutinib (crystal form A) | 80.00 mg |
|---|---|
| Microcrystalline cellulose | 260.20 mg |
| Croscarmellose sodium | 14.40 mg |
| Colloidal silicon dioxide | 1.8 mg |
| Sodium dodecyl sulfate | 1.8 mg |
| Magnesium stearate | 1.8 mg |
| Total amount | 360 mg |

A target oral capsule was prepared with reference to a method similar to that in Example 1. The cumulative dissolution rate (in vitro dissolution) of the drug was determined with reference to the method in Example 1. The oral zanubrutinib capsule in Example 3 has a dissolution rate (%)of more than 85% in the above dissolution medium in 30 min.

### Example 4

### Preparation of oral zanubrutinib capsule

Formula for a single capsule:

| Zanubrutinib (crystal form A) | 80.00 mg |
|---|---|
| Microcriystalline cellulose | 263.80 mg |
| Croscarmellose sodium | 10.80 mg |
| Colloidal silicon dioxide | 1.8 mg |
| Sodium dodecyl sulfate | 1.8 mg |
| Magnesium stearate | 1.8 mg |
| Total amount | 360 mg |

A target oral capsule was prepared with reference to a method similar to that in Example 1. The cumulative dissolution rate (in vitro dissolution) of the drug was determined with reference to the method in Example 1. The oral zanubrutinib capsule in Example 4 has a dissolution rate (%)of more than 85% in the above dissolution medium in 30 min.

### Example 5

### Preparation of oral zanubrutinib capsule

Formula for a single capsule:

| Zanubrutinib (crystal form A) | 80.00 mg |
|---|---|
| Microcrystalline cellulose | 262.00 mg |
| Croscarmellose sodium | 10.80 mg |
| Colloidal silicon dioxide | 1.8 mg |
| Sodium dodecyl sulfate | 1.8 mg |
| Magnesium stearate | 3.60 mg |
| Total amount | 360 mg |

A target oral capsule was prepared with reference to a method similar to that in Example 1. The cumulative dissolution rate (in vitro dissolution) of the drug was determined with reference to the method in Example 1. The oral zanubrutinib capsule in **Example 5** has a dissolution rate (%)of more than 85% in the above dissolution medium in 30 min.

### Example 6

### Preparation of oral zanubrutinib capsule

Formula for a single capsule:

| Zanubrutinib (crystal form A) | 80.00 mg |
|---|---|
| Microcrystalline cellulose | 258.40 mg |
| Croscarmellose sodium | 10.80 mg |
| Colloidal silicon dioxide | 1.8 mg |
| Sodium dodecyl sulfate | 1.8 mg |
| Magnesium stearate | 7.20 mg |
| Total amount | 360 mg |

A target oral capsule was prepared with reference to a method similar to that in Example 1. The cumulative dissolution rate (in vitro dissolution) of the drug was determined with reference to the method in Example 1. The oral zanubrutinib capsule in **Example 6** has a dissolution rate (%)of more than 85% in the above dissolution medium in 30 min.

The present disclosure has been described in detail above with general description, detailed description, and experiments. Modifications or improvements made without departing from the spirit of the present disclosure fall within the scope of protection of the present disclosure.

## Claims

1. An oral capsule comprising zanubrutinib, the oral capsule comprising a composition for the oral capsule and a capsule shell, the composition for the oral capsule comprising zanubrutinib, a filler, a disintegrant, a wetting agent, a glidant, and a lubricant.

2. An oral capsule comprising zanubrutinib, a composition for the oral capsule further comprising an optional binder.

3. The oral capsule according to claim 1 or 2, wherein the zanubrutinib is a crystal form A or an amorphous form or a mixture of a crystal form A and an amorphous form, a particle size of the zanubrutinib is less than or equal to 40 µm, and a mass percent of the zanubrutinib is from 20% to 70%, preferably from 20% to 50%, relative to a total mass of the composition for the oral capsule.

4. The oral capsule according to any one of claims 1 to 3, wherein the filler is selected from the group consisting of starch, sucrose, microcrystalline cellulose, anhydrous calcium hydrogen phosphate, mannitol, lactose, pregelatinized starch, glucose, maltodextrin, cyclodextrin, cellulose, silicified microcrystalline cellulose, and any combination thereof; and a mass percent of the filler is from 20% to 90%, preferably from 30% to 80%, relative to the total mass of the composition for the oral capsule.

5. The oral capsule according to claim 4, wherein the filler is microcrystalline cellulose; and a mass percent of the microcrystalline cellulose is from 30% to 80% relative to the total mass of the composition for the oral capsule.

6. The oral capsule according to any one of claims 1 to 3, wherein the disintegrant is selected from the group consisting of sodium carboxymethyl starch, low-substituted hydroxypropylcellulose, crospovidone, croscarmellose sodium, croscarmellose, methylcellulose, pregelatinized starch, sodium alginate, and any combination thereof, and is preferably croscarmellose sodium; and a mass percent of the disintegrant is from 0.5% to 5%, preferably from 1% to 3%, relative to the total mass of the composition for the oral capsule.

7. The oral capsule according to any one of claims 1 to 3, wherein the wetting agent is sodium dodecyl sulfate; and a mass percent of the sodium dodecyl sulfate is from 0% to 5%, preferably from 0.5% to 1.0%, relative to the total mass of the composition for the oral capsule.

8. The oral capsule according to any one of claims 1 to 3, wherein the glidant is selected from the group consisting of powdery cellulose, magnesium trisilicate, colloidal silicon dioxide, talcum powder, and any combination thereof, and is preferably colloidal silicon dioxide; and a mass percent of the glidant is from 0.1% to 20%, preferably from 0.1% to 0.5%, relative to the total mass of the composition for the oral capsule.

9. The oral capsule according to any one of claims 1 to 3, wherein the lubricant is selected from the group consisting of zinc stearate, glyceryl monostearate, glyceryl palmitostearate, magnesium stearate, sodium stearyl fumarate, and any combination thereof, and is preferably magnesium stearate; and a mass percent of the lubricant is from 0.1% to 2%, preferably from 0.3% to 1%, relative to the total mass of the composition for the oral capsule.

10. The oral capsule according to any one of claims 1 to 3, wherein the capsule shell is a gelatin capsule shell.

11. A method for preparing the oral capsule according to any one of claims 1 to 10, comprising following steps:
(1) pre-mixing zanubrutinib, a disintegrant, a wetting agent and a part of a filler to obtain a premix, and then sieving the premix to obtain a first mixture;
(2) sieving a glidant and the remaining part of the filler, and adding the sieved materials to the first mixture obtained in step (1) and mixing to obtain a second mixture;
(3) sieving a lubricant, adding the sieved lubricant to the second mixture obtained in step (2), and then mixing to obtain a final mixture; and
(4) encapsulating the final mixture obtained in step (3) into a capsule shell to obtain the oral capsule.

12. The method according to claim 11, wherein the pre-mixing in step (1) is performed at a revolving speed from 10 rpm to 25 rpm for 2 min to 10 min, and a sieve used for the sieving has a mesh size from 1.0 mm to 2.5 mm.

13. The method according to claim 12, wherein the first mixture in step (1) is obtained by mixing the premix at a revolving speed from 10 rpm to 25 rpm for 40 min to 15 min.

14. The method according to any one of claims 11 to 13, wherein the mixing in step (2) is performed at a revolving speed from 10 rpm to 20 rpm for 3 min to 5 min.

15. The method according to any one of claims 11 to 14, wherein the sieving in step (2) is performed at a revolving speed from 550 rpm to 650 rpm, and a sieve used for the sieving has a mesh size from 1.0 mm to 2.5 mm.

16. The method according to any one of claims 11 to 15, wherein the mixing in step (3) is performed at a revolving speed from 10 rpm to 15 rpm for 3 min to 6 min, and a sieve used for the sieving has a mesh size from 35 mm to 45 mm.

17. The method according to any one of claims 11 to 16, wherein a loading amount of a mixing hopper is from 30% to 70% of the volume of the mixing hopper.
